# EUROPÄISCHE PATENTANMELDUNG

(11) **EP 3 335 761 A1**
(43) Veröffentlichungstag der Anmeldung: **20.06.2018**
(21) Anmeldenummer: 17201332.8
(22) Anmeldetag: 13.11.2017
(51) Int. Cl.: A61N 5/06

(54) **SOFT-LASER ZUR BEHANDLUNG VON BESCHWERDEN**

(30) Priorität: 16.12.2016 CH 16742016
(71) Anmelder: Sardo, Flavio, 8118 Pfaffhausen (CH)
(72) Erfinder: Sardo, Flavio, 8118 Pfaffhausen (CH)
(74) Vertreter: Felber, Josef

(57) **Zusammenfassung**

Der Soft-Laser besteht aus einem Gehäuse (1) zur Aufnahme des Lasers und der Batterien mit einem an einem Ende des Gehäuses (1) angedockten Lichtleiter in Form einer Fiberglasfaser (4). Die Fiberglasfaser (4) ist in einem krümmbaren flexiblen Schlauch (3) geführt, welcher mittels eines Adapters (2) mit dem Gehäuse (1) verbindbar ist. An seinem freien Ende ist ein Einsatzteil (6) über den Flex-Schlauch (3) steckbar, durch welches die Fiberglasfaser (4) geführt ist. Am Ende trägt die Fiberglasfaser (4) Lichtstreukugel (8), wozu diese eine Sackbohrung aufweist, in welche die Fiberglasfaser (4) gesteckt ist. Die Aussenfläche der Lichtstreukugel (8) bleibt einen Raumwinkel von idealerweise 300° frei und somit wird das Laserlicht in allen Richtungen innerhalb dieses Winkels Laserlicht ausgestrahlt.

## Beschreibung

Von Tinnitus aurium oder kurz Tinnitus spricht man, wenn eine Person ständig oder phasenweise ein Pfeifen, Klingeln, Rauschen, Sausen oder ein Brummen hört. Im Volksmund spricht man auch von Ohrensausen oder Ohrenrauschen. Symptome von Tinnitus und ähnlichen Innenohrerkrankungen sind Schwerhörigkeit, Druck im Ohr, Schwindel (Morbus Meniere, Vertigo), Hörsturz und Hörverzerrung. Jeder Tinnitus ist individuell und jeder sollte daher individuell behandelt werden, denn Tinnitus beeinträchtigt die Lebensqualität erheblich. Er reduziert die Lebensfreude, zumal oftmals wenig oder keine Hoffnung auf Besserung in Aussicht steht. In Zusammenarbeit mit Betroffenen und Fachleuten werden die verschiedenen Tinnitusse immer weiter erforscht und es werden neue Therapien entwickelt.

Einer neueren Studie zufolge erlebte fast ein Viertel der Bevölkerung schon einmal ein Ohrensausen. Fast 10% der Bevölkerung hören ab und zu solche Ohrgeräusche. Und 3% der Bevölkerung leiden an einem chronischen Tinnitus, ein Ohrgeräusch, das sie ständig begleitet. Es hat sich gezeigt, dass je früher eine Therapie begonnen wird, umso eher sich ein Erfolg einstellt. In 99% aller Fälle hat ein Tinnitus eine gutartige Ursache. Er kann als ein natürliches Risiko des modernen Lebens und Teil des Alterungsprozesses betrachtet werden.

Es gibt eine Reihe erfolgreicher Behandlungsmöglichkeiten, welche ihre Berechtigung bei ganz bestimmten Symptom-Mustern haben. Die Schwierigkeit bei einer Tinnitustherapie besteht jedoch generell darin, die richtige individuelle Therapie für ein ganz spezifisches Krankheitsbild zu finden.

Die Softlaser-Technologie erweist sich als wirksame Therapie gegen Tinnitus aurium (Ohrensausen), um namentlich Innenohrerkrankungen wie Tinnitus-bedingte Schwerhörigkeit, Druck Im Ohr, Morbus Meniere (Schwindel), Hörsturz und Hörverzerrung durch die betroffene Person selbst zu behandeln. Aber auch andere Beschwerden, etwa Polypen in der Nase oder Aphten im Mund, lassen sich erfolgreich mit einem Soft-Laser therapieren. Ein solches Softlaser-System besteht aus einem Laser mit 5mW Leistung und einer Wellenlänge von 650nm. Eine Fiberglasfaser als ein Lichtleiter führt den Laser ins Innere des Ohrs, des Mundes oder der Nase und der Laserstrahl trifft dort auf die zu behandelnde Stelle. Dieses Gerät bietet den überzeugenden Vorteil, dass die Therapie vom Patienten selbst und dies ortsabhängig, das heisst auch zuhause, durchgeführt werden kann. Dieser Soft-Laser wird oftmals zur Begleitung einer professionellen Therapie eingesetzt, und gerade dieser Einsatz erweist sich als besonders wirksam. Die Behandlung mit einem solchen Soft-Laser ist völlig sicher und schmerzlos. Der Laserstrahl durchdringt selbst tiefere Unterhautschichten und wirkt so als heilsame Biostimulation direkt auf den Stoffwechsel im Bindegewebe. Das führt zur rasanten Regeneration zum Beispiel der Hörzellen, zur Stimulation des Immunsystem, zur Anregung der Zellteilung und zur Aktivierung bestimmter Abwehrmoleküle.

Viele Krankheiten entstehen durch einen Mangel an lebensnotwendiger Zell-Energie, genannt **A**denosin**T**ri**P**hosphat ATP, eine Schlüsselsubstenz unserer Körperzellen. Die Verbrennung von Traubenzucker in den Kraftwerken der Zellen, den sogenannten Mitochondrien, dient dazu, genügend Energie für die energieverbrauchenden Prozesse in den Zellen bereit zu stellen. Ist die ATP-Versorgung ungenügend, führt dies in der Folge zur Schädigung und schliesslich zum Tod der Zellen. Das konzentrierte Licht des Soft-Lasers mit seiner Wellenlänge von 650 nm stimuliert in den bestrahlten Zellen die Zuckerverbrennung und verbessert somit die ATP-Versorgung der Zellen. Die Regeneration geschädigter Hör-Sinneszellen kann mit so einem Softlaser beschleunigt werden. Ausserdem können durch Stimulierung der gesunden Zellen Aphten und Polypen therapiert werden.

Bei Tinnitus wird eine einmalige Behandlung pro Tag über eine Zeitperiode von 20 Minuten empfohlen, und dies über mindestens 10 Wochen hinweg. Nur diese regelmässige Anwendung des Soft-Lasers erbringt die gewünschten Behandlungserfolge. Die Betriebstemperatur liegt bei bis zu 43°C. Der Soft-Laser wird mit zwei 1.5V Batterien betrieben, welche zusammen eine Kapazität von ca. 20 Stunden erbringen. Nach jeder Behandlung wird das Gerät gereinigt, vor allem wenn verschiedene Anwender das gleiche Gerät benützen. Die abschraubbaren Zuhörteile können mit einem Desinfektionsmittel gereinigt oder in kochendes Wasser gelegt oder bei maximal 135° C sterilisiert werden. Mit diesem System konnten bereits über 40'000 Betroffene erfolgreich behandelt werden.

Nach dem Prinzip des Soft-Lasers wird mittels einer ausgeklügelten Glasfaserleitung die benötigte Energie konzentriert und exakt auf die erkrankte Stelle projiziert. Dabei wir eine grosse Wirkungsqualität bei gleichzeitig höchst einfacher Bedienung erreicht. Die Anwendung des Softlasers kann vorteilhaft mit einer Klangtherapie kombiniert werden. Diese Laserbehandlung ist inzwischen in Kliniken und Arztpraxen etabliert und seit langer Zeit erprobt. Es wurden umfangreiche medizinische Tests und diverse medizinische Studien durchgeführt und die Ergebnisse sind wissenschaftlich belegt. Die Sicherheit und Wirkung des Softlasers wurde attestiert und medizinisch zertifiziert. Der Soft-Laser ist ausserdem mit einem CE-Zeichen geprüft. Er entspricht den einschlägigen Bestimmungen der EG-Richtlinie 93/42/EWG und die Qualitätsmassnahmen erfüllen die Anforderungen nach dem Anhang VII und V dieser EG-Richtlinie 93/42/EWG. Als Medizinprodukt entspricht dieser Soft-Laser ausserdem den Anforderungen nach den Europäischen Normen EN 60601.1, EN60601-1-2 und EN 60825-1. Dazu wird auch die Ergänzungsnorm erfüllt, nämlich die elektromagnetische Verträglichkeit mit ihren Anforderungen und Prüfungen. Der Soft-Laser hat keinen Einfluss auf andere Geräte in seiner Nähe. Letztlich verfügt er über die Zertifizierung CE (Europa) 93-42 EEC für Europa, dann CB (International) IEC-EN 60601 und ETL (USA/Kanada) US 6060. Ein solcher Soft-Laser ist in WO2007/019720 A1 ausführlich offenbart und beschrieben. Dieser dort offenbarte Laser bietet eine Leistung von 5mW und gehört zur Laserklasse 1 als Gefahrenklasse, darf also an nicht fachkundige Personen ausgehändigt werden.

Ausgehend von diesem Stand der Technik besteht die Aufgabe dieser vorliegenden Erfindung darin, einen Soft-Laser weiterzuentwickeln und namentlich eine wesentlich höhere Leistung zu ermöglichen, wobei dieser Soft-Laser aber immer noch in der Laserklasse 1 als Gefahrenklasse fallen soll, womit er an nicht fachkundige Personen abgegeben werden darf. Ausserdem ist es eine Aufgabe der Erfindung, den Soft-Laser so zu gestalten, dass das angestrahlte Laserlicht auf schwer zugängliche Stellen in Körperöffnungen, etwa in der Nase, im Mund oder im Innenrohr richtbar ist.

Diese Aufgabe wird gelöst von einem Soft-Laser, bestehend aus einem Gehäuse zur Aufnahme des Lasers und der Batterien mit einem an einem Ende des Gehäuses angedockten Lichtleiter in Form einer Fiberglasfaser, dadurch gekennzeichnet, dass die Fiberglasfaser in einem krümmbaren Flex-Schlauch geführt ist, welcher mittels eines Adapters mit dem Gehäuse fest verbunden ist, und der an seinem freien Ende an ein Einsatzteil steckbar ist, durch welches die Fiberglasfaser geführt ist, die mit ihrem Ende eine Lichtstreukugel trägt, welche eine Sackbohrung aufweist, in welche die Fiberglasfaser gesteckt ist, sodass die Lichtstreukugel über einen Raumwinkel von wenigstens 180° frei bleibt und somit in allen Richtungen innerhalb dieses Raumwinkels Laserlicht ausstrahlbar ist.

In den Figuren sind ein Ausführungsbeispiel und die einzelnen Bestandteile eines solchen Soft-Lasers dargestellt und seine Bestandteile werden im Folgenden beschrieben. Diese Bestandteile sind dabei aber nicht im gleichen Massstab dargestellt.

Es zeigt:
- Figur 1:: Den Soft-Laser mit Gehäuse und dem daran angeschlossenen Schlauch und der darin geführten Fiberglasfaser;
- Figur 2:: Den Soft-Laser mit Gehäuse, und in einem Längsschnitt den daran angeschlossenen Schlauch und die darin geführte Fiberglasfaser mit ihrer besonderen Lichtstreukugel am vorderen Ende;
- Figur 3:: Den Adapter zum Verbinden des krümmbaren Schlauches mit dem Gehäuse des Lasers in perspektivischer Ansicht dargestellt;
- Figur 4:: Den Adapter zum Verbinden des krümmbaren Schlauches mit dem Gehäuse des Lasers in einem axialen Längsschnitt dargestellt;
- Figur 5:: Die Klemm-Muffe zum Sichern des Schlauches auf dem Adapter in einem axialen Längsschnitt dargestellt;

- Figur 6:: Den flexiblen Schlauch;
- Figur 7:: Den flexiblen Schlauch in gekrümmter Lage;
- Figur 8:: Das Einsatzteil für das vordere Ende des Schlauches mit Fassung für die Lichtstreu kugel;
- Figur 9:: Das Einsatzteil für das vordere Ende des Schlauches mit Fassung für die Lichtstreukugel in einem axialen Längsschnitt dargestellt;
- Figur 10:: Die Lichtstreukugel in einem Längsschnitt längs der Achse ihrer Sackbohrung dargestellt;
- Figur 11:: Eine Schutzkappe für das Einsatzteil;
- Figur 12:: Die Schutzkappe für das Einsatzteil nach Figur 11 in einem axialen Längsschitt dargestellt;
- Figur 13:: Eine weitere Kappe für das Einsatzteil als Ständer, in einem axialen Längsschnitt dargestellt;
- Figur 14:: Die Kappe als Ständer nach Figur 13, in Perspektive;
- Figur 15:: Eine hutförmige Kappe für das Einsatzteil, in einem axialen Längsschnitt dargestellt;
- Figur 16:: Die Kappe nach Figur 15 in Perspektive;
- Figur 17:: Eine weitere Kappe zum Schützen der Lichtstreukugel in einem axialen Längsschnitt dargestellt;
- Figur 18:: Die Kappe nach Figur 17 von hinten gesehen.

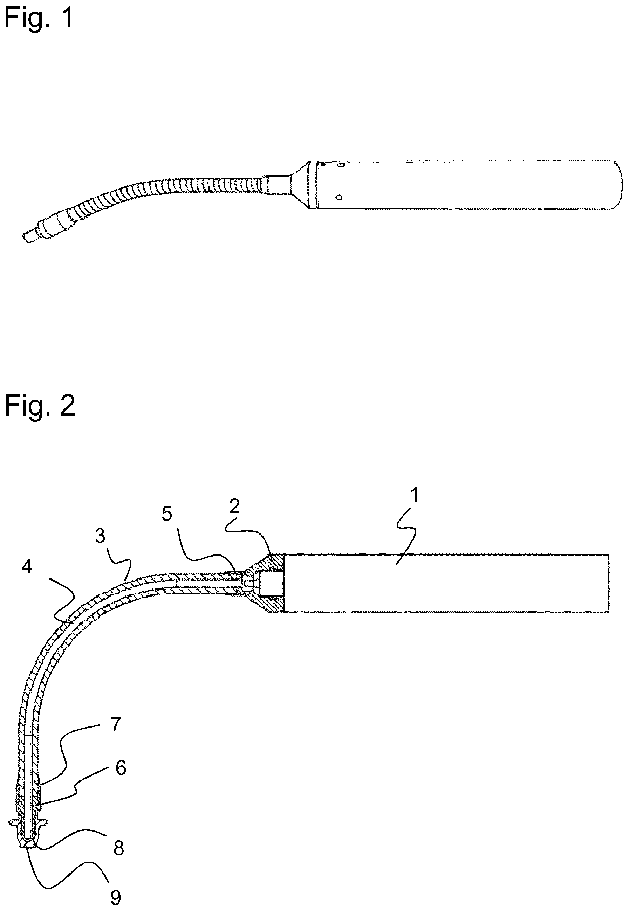

Die Figur 1 zeigt den gesamten Soft-Laser. Im Gehäuse 1 sind der eigentliche Laser und seine Energieversorgung untergebracht. Die Energieversorgung ist durch zwei 1.5V Batterien sichergestellt. Die aussen am Gehäuse sind anzuschliessenden Komponenten für die Führung des Lasers angebaut, hier in Form eines flexiblen Schlauch, welcher eine Fiberglas-Faser für die Führung des Lasers bis an sein Ende führt.

Die Figur 2 zeigt diesen Soft-Laser ab dem Gehäuse in einem Längsschnitt dargestellt. Nach dem Gehäuse 1 folgt zunächst ein Adapter 2 zum Verbinden eines flexiblen Schlauches 3 mit dem Gehäuse 1. Im Innern des Schlauches 3 ist die Fiberglasfaser 4 geführt, zur verlustarmen Lichtleitung des Lasers an ihr vorderes Ende. Der krümmbare bzw. flexible Schlauch 3 lässt sich über den Adapter 2 schieben und mit einer Klemm-Muffe 5 sichern. Diese wird von Schlauch 3 her über den am Adapter 2 vorhandenen Nippel geschoben und verklemmt den Schlauch 3 darauf. Am vorderen Ende des Schlauches 3 erkennt man ein Einsatzteil 6, das gegen den Schlauch 3 hin in einen Nippel ausgeformt ist, über den der Schlauch 3 gesteckt ist. Vom Schlauch 3 her kann wiederum eine Klemm-Muffe 7 über das Einsatzteil 6 geschoben werden, zur Sicherung des Schlauches auf dem Einsatzteil 6. Vorne im Einsatzteil 6 bildet dieses als Besonderheit eine sphärische Fassung zur Aufnahme einer Licht-Streukugel 8, welche darin einen passgenauen Sitz erhält. Über diese Licht-Streukugel 9 und das vordere Einsatzteil 6 ist bei Nichtgebrauch des Soft-Lasers eine Schutzkappe 9 aufsteckbar.

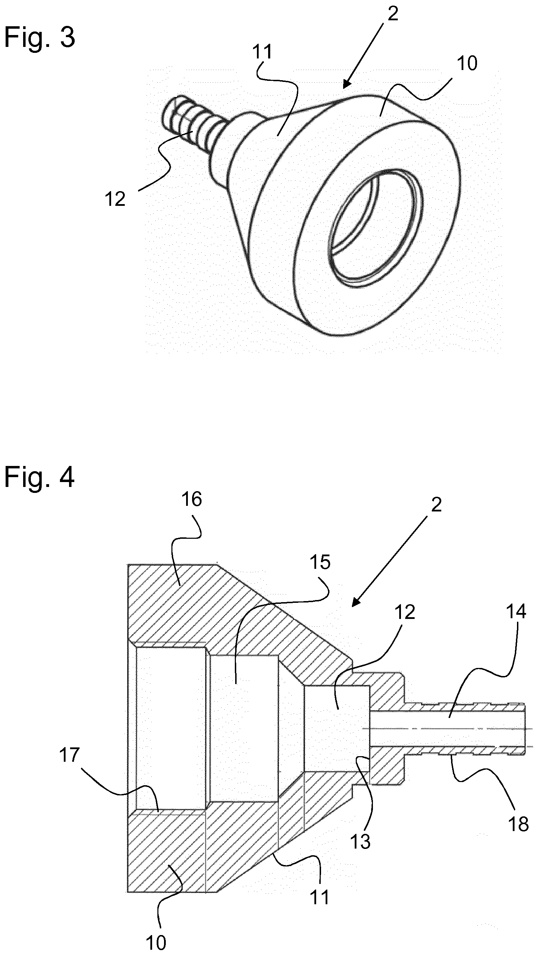

Die Figur 3 zeigt den Adapter 2 zum Verbinden des krümmbaren Schlauches 3 mit dem Gehäuse 1 des Lasers gesondert, in einer perspektivischen Ansicht dargestellt. Man sieht ihn hier von der Seite des anzuschliessenden Gehäuses 1 her gesehen. Er formt zunächst einen Hohlzylinder 10, der dann in einen Konus 11 übergeht, und vorne in einen Nippel 12 ausläuft, über den der krümmbare Schlauch 3 steckbar ist.

Die Figur 4 zeigt diesen Adapter 2 in einem axialen Längsschnitt dargestellt. In seinem Innern gibt es eine Kaskade von Sackbohrungen. Jene Bohrung 12 mit dem geringsten Durchmesser reicht in den Konus 11 hinein und leicht über dessen Ende hinaus und ist dann von ihrem Boden 13 aus in einer weiteren, dünneren Bohrung 14 axial weitergeführt, sodass diese dünnere Bohrung 14 gerade die Fiberglasfaser zum Führen des Laserlichts satt passend aufnimmt. In die andere Richtung folgt nach der dünnsten Sackbohrung 12 eine nächstgrössere Bohrung 15, und schliesslich eine noch etwas grössere Bohrung 16 mit Innengewinde 17 der Dimension M10x0.5. Damit lässt sich der Adapter 2 auf ein entsprechendes Aussengewinde am Gehäuse 1 aufschrauben und somit satt und fest mit dem Gehäuse 1 verbinden. Am vorderen Ende, vor dem zugelaufenen Konus 11 erstreckt sich der Nippel 18, dessen Aussenseite abwechselnd leicht unterschiedliche Aussendurchmesser aufweist, oder als Alternative eine Riffelung, um die Reibkräfte zu vergrössern, wenn ein Schlauch 3 über diesen Nippel 18 geschoben wird.

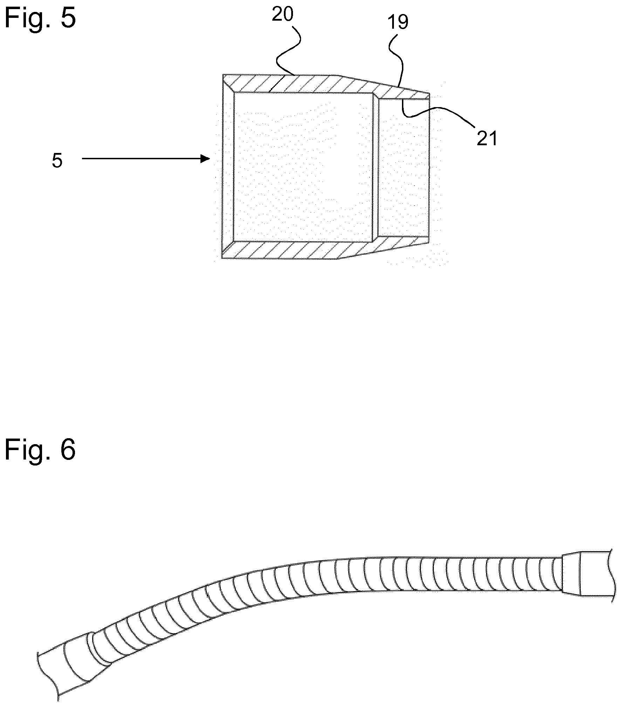

Die Figur 5 zeigt eine Klemm-Muffe zum Sichern des Schlauches 3 auf dem Adapter 2 in einem axialen Längsschnitt dargestellt. Sie weist im gezeigten Beispiel eine Länge von 9mm auf und zunächst einen Innendurchmesser von 6.5mm, und hinten, das heisst im Bild rechts läuft sie aussen konisch über den hintersten Abschnitt 19 zu, wobei sie dort einen leicht reduzierten Innendurchmesser von 6mm aufweist. Damit kann sie mit dem grösseren Innendurchmesser 20 voraus über den Schlauch 3 geschoben werden und schliesslich über den auf den Nippel 18 gesteckten Schlauch 3, sodass sich der kleinere Innendurchmesser 21 klemmend über den Schlauch 3 und Nippel 18 schiebt und eine sichere Klemmverbindung zwischen Schlauch 3 und Nippel 18 sicherstellt.

Die Figur 6 zeigt den krümmbaren flexiblen Schlauch 3. Er kann zu seinem Schutz wie gezeigt von einem Mantel wie ein miniaturisierter Duschschlauch eingefasst sein. Im Innern besteht er aus einem flexiblen Gummischlauch, in dessen Innerem die Fiberglasfaser 4 als Leiterin des Laserlichtes satt von ihm gehalten geführt ist.

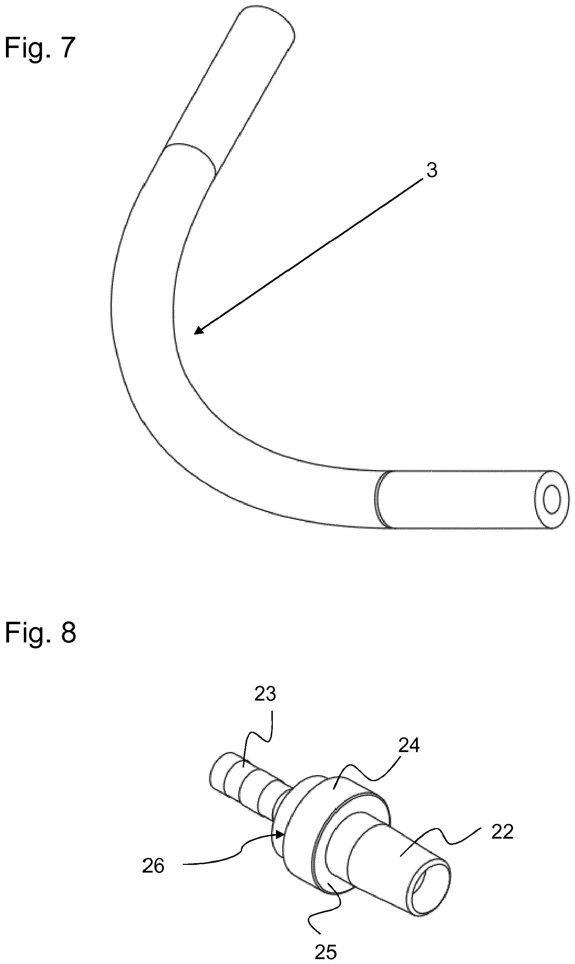

Wie in Figur 7 gezeigt, kann der krümmbare flexible Schlauch 3 ohne Weiteres um einen Winkel von 90° gekrümmt werden, wobei diese Krümmung aus der Geraden bis hin zu diesem 90°-Winkel jeden Zwischenwinkel stabil bleibend einnehmen kann. Damit wird sichergestellt, dass man in jeder Lage das vordere Ende des Schlauches 3 und sein Einsatzstück 6 an die gewünschte Körperstelle führen kann, zum Beispiel in das Ohr, in die Nase oder den Mund, entweder selbst oder durch eine Hilfsperson.

Die Figur 8 zeigt das Einsatzteil für das vordere Ende des Schlauches 3 mit seiner Fassung für die Lichtstreukugel in einer perspektivischen Ansicht dargestellt. Es bildet vorne eine dickeres Rohr 22, und hinten einen dünneres, das als Nippel 23 ausgebildet ist, und dazwischen einen verdickten Abschnitt 24 zur Bildung je einer in axialer Richtung wirkenden Schulter 25,26.

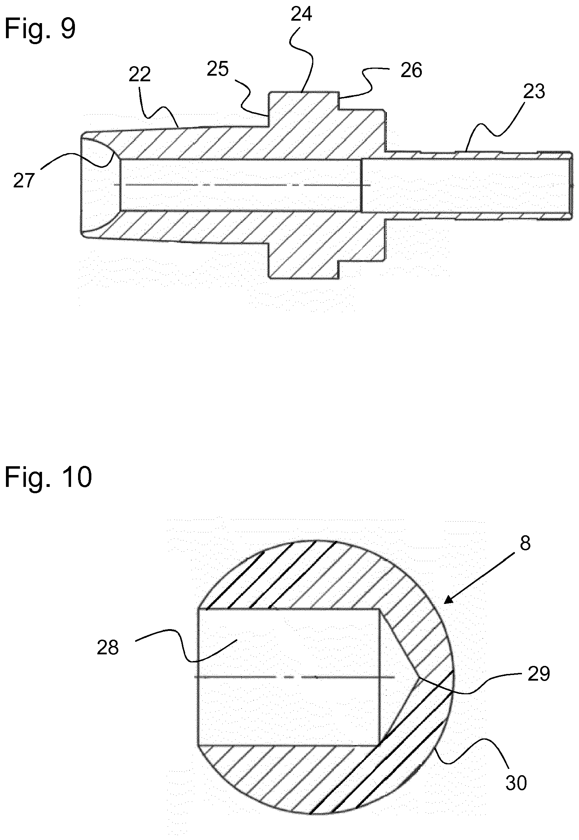

Die Figur 9 zeigt diese Einsatzteil für das vordere Ende des Schlauches mit Fassung für die besondere Lichtstreukugel in einem axialen Längsschnitt dargestellt. Das vordere Ende 22 liegt hier links und es bildet dort eine halbe sphärische Einsatzfläche 27 zum Aufnehmen einer dazu passenden Licht-Streukugel. Weiter gegen hinten folgt nach einem ca. 8mm langen Stück 22 eine Verdickung 24 des Aussendurchmessers, sodass in axialer Richtung auf beiden Seiten dieser Verdickung 24 je eine Schulter 25,26 gebildet ist. Ein stückweit nach der hinteren Schulter 27 folgt eine Reduktion des Aussendurchmesser, wodurch ein Nippel 23 gebildet ist, welcher ebenfalls eine geriffelte oder sonst wie gestaltete Umfangsfläche aufweist, sodass ein Schlauch 3 mit verstärkter Reibung darauf schiebbar ist. Der Schlauch 3 kann auf diesem Nippel 23 wiederum mit einer Klemm-Muffe 7 gesichert werden, wie schon am Nippel 12 des Adapters 2.

Die Figur 10 zeigt das Kernstück dieses vorliegenden Soft-Lasers, nämlich eine besondere Lichtstreukugel 8. Diese besteht aus einem transparenten Kunststoff, zum Beispiel aus PA oder aus demselben Material wie die Fiberglasfaser 4, die als Lichtleiter wirkt. Diese Kugel weist einen doppelten Durchmesser wie die Fiberglasfaser auf und von einer Seite her ist eine Sackbohrung 28 von exakt dem Durchmesser der Fiberglasfaser 4 in sie hineingebohrt, und wie im gezeigten Beispiel über eine Tiefe von 3mm, was die Bohrspitze 29 betrifft. Die Fiberglasfaser 4 lässt sich daher in diese Sackbohrung hineinschieben, und das von ihr vorne austretende Laserlicht wird von der Lichtstreukugel 8 diffus in alle Richtungen gestreut und an der Umfangsfläche 30 gebrochen. Laserlicht wird in alle Richtung von der Kugel 8 abgestrahlt, in einem Raumwinkel von etwa 300 Grad. Gerade diese Massnahme erbringt den Vorteil, dass beim Bestrahlen des Körperteils der Lichtstrahl nicht bloss in axialer Richtung der Fiberglasfaser 4 auf eine bestimmte Stelle gerichtet auftrifft, sondern in diesem Raumwinkel rundum auf das Innern des Ohres, Nase, Mund auftrifft und dort seine Wirkung entfaltet. Weil die Leistung des Lasers über diesen grossen Raumwinkel gestreut wird, können weit stärkere Laser von zum Beispiel 100mW eingesetzt werden, und dennoch vermag das Gerät unter die Leistungsklasse 1 zu fallen, denn die Lichtintensität des an einer bestimmten Stelle im Innern des Ohres, Nase, Mund auftreffenden Laser-Lichtes ist nicht grösser als bei axialer direkter Bestrahlung mit einem 5mW Laser.

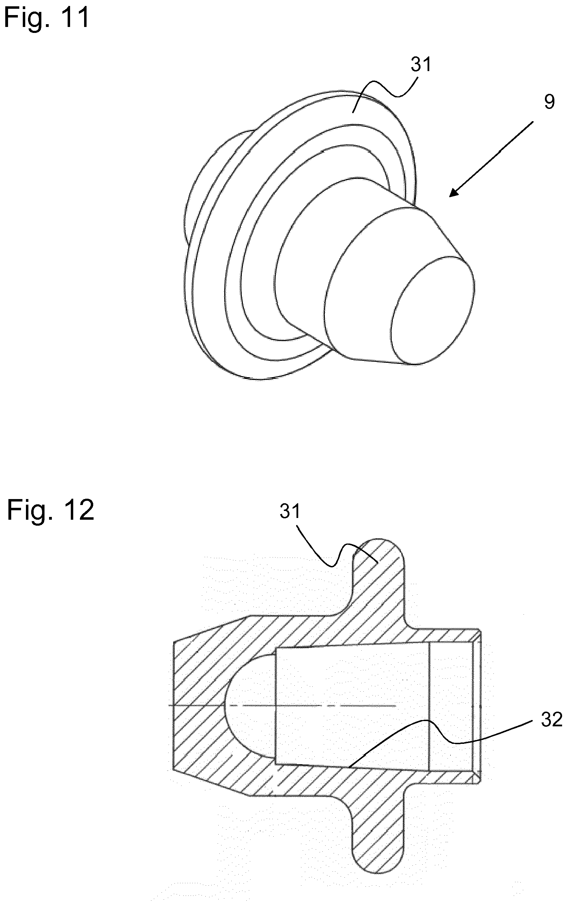

Wenn der Soft-Laser nicht gebraucht wird, kann eine Schutzkappe 9 wie in Figur 11 über sein vorderes Ende, das heisst über seine Lichtstreu-Kugel 8 gestülpt werden, damit diese keinen Schaden nehmen kann. Diese Schutzkappe 9 ist in Figur 12 noch in einem axialen Schnitt dargestellt. Sie passt mit ihrem Innern genau über das Einsatzteil 6 und die vorne in diesem sitzenden Lichtstreu-Kugel 8. Sowohl das Einsatzteil 6 wie auch die Innenfläche 32 der Schutzkappe verlaufen ganz leicht konisch, sodass beim Aufsetzen der Schutzkappe 9 auf das Einsatzteil 6 eine Klemmwirkung entsteht. Aussen weist sie einen auskragenden Ring 31 auf, an dem sie leicht ergriffen werden kann.

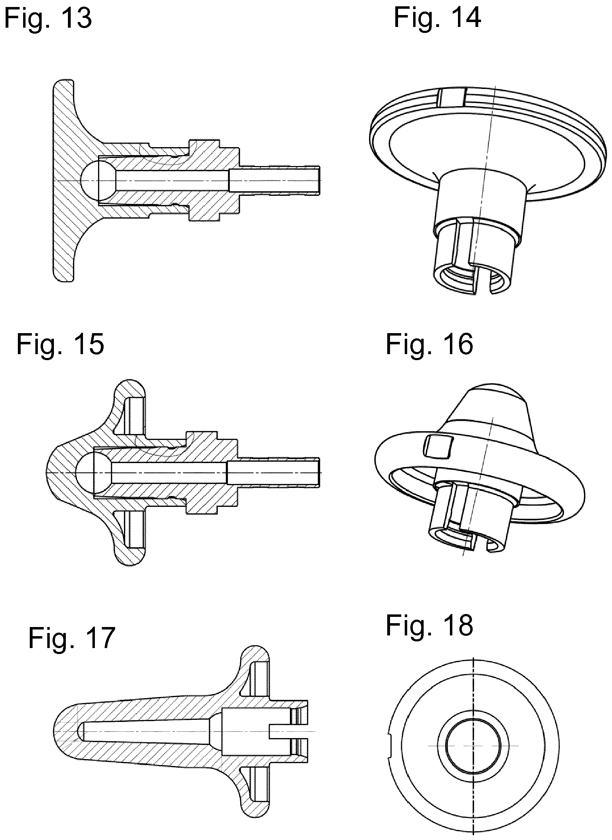

Die Figuren 13 bis 18 zeigen weitere alternative Ausführungen solcher Schutzkappen. In Figuren 13 und 14 ist eine solche Schutzkappe als Ständer ausgebildet, indem sie einen tellerförmigen Boden bildet, auf den sie abgestellt werden kann. Der Soft-Laser kann dann mit seinem Einsatzteil und getreckten Schlauch in diese Schutzkappe gesteckt werden und platzsparend versorgt werden. Die Figuren 15 und 16 zeigen eine Schutzkappe mit hutförmiger Ausgestaltung, und die Figuren 17 und 18 eine weitere Schutzkappe zum Einstecken des vorersten Teils des Soft-Lasers mit seiner Streukugel.

## Patentansprüche

1. Soft-Laser, bestehend aus einem Gehäuse (1) zur Aufnahme des Lasers und der Batterien mit einem an einem Ende des Gehäuses (1) angedockten Lichtleiter in Form einer Fiberglasfaser (4), ***dadurch gekennzeichnet,* dass** die Fiberglasfaser (4) in einem flexiblen Schlauch (3) geführt ist, welcher mittels eines Adapters (2) mit dem Gehäuse (1) fix verbindbar ist, und der an seinem freien Ende über ein Einsatzteil (6) steckbar ist, durch welches die Fiberglasfaser (4) geführt ist, die mit ihrem Ende eine Lichtstreukugel (8) trägt, welche eine Sackbohrung (28) aufweist, in welche die Fiberglasfaser (4) gesteckt ist, sodass die Aussenfläche (30) der Lichtstreukugel (8) über einen Raumwinkel von wenigstens 180° frei bleibt und somit in allen Richtungen innerhalb dieses Raumwinkels Laserlicht ausstrahlbar ist.

2. Soft-Laser nach Anspruch 1, ***dadurch gekennzeichnet,* dass** der flexible Schlauch (3) wenigstens 60mm Länge misst, und die Lichtstreukugel (8) am vorderen Ende der Fiberglasfaser (4) von doppeltem Durchmesser ist wie die Fiberglasfaser (4) und eine Sackbohrung (28) vom Durchmesser der Fiberglasfaser (4) aufweist und eine Tiefe über das Zentrum der Lichtstreukugel (8) hinaus aufweist, sodass die Aussenfläche (30) der Lichtstreukugel (8) über einen Raumwinkel von 300° frei bleibt und somit in allen Richtungen innerhalb dieses Winkels Laserlicht ausstrahlbar ist.

3. Soft-Laser nach einem der vorangehenden Ansprüche, ***dadurch gekennzeichnet,* dass** die Fiberglasfaser (4) im Durchmesser 2mm misst und der flexible Schlauch (3) einen Innendurchmesser von 2mm aufweist und einen Aussendurchmesser von 5-6mm, sowie eine Länge im geraden Zustand von 6cm bis 9cm, und dass der Adapter (2) zur Verbindung der Fiberglasfaser (4) und des flexiblen Schlauches (3) mit dem Gehäuse (1) eine Muffe bildet, welche von gleichem Durchmesser wie das Gehäuse (1) misst und in Richtung weg vom Gehäuse (1) in einen konisch zulaufenden Abschnitt (11) ausläuft, innerhalb welches Abschnittes (11) der Adapter (2) zunächst ein Innengewinde (17) aufweist und hernach eine Axialbohrung (15) mit kleinerem Durchmesser als das Innengewinde (17), gefolgt von einer weiteren Axialbohrung (12) mit abermals kleinerem Durchmesser, an welche eine Bohrung von 2mm Durchmesser zur Durchführung der Fiberglasfaser (4) anschliesst, und welche auf ihrer Aussenseite einen geriffelten Nippel (18) mit Aussendurchmesser 3mm bildet, auf welchen der flexible Schlauch (3) klemmend aufsteckbar ist, wobei über die Steckverbindung eine Klemm-Muffe (5) mit Innendurchmesser 6.5mm schiebbar, die gegen die Seite des herausragenden flexiblen Schlauches (3) hin aussen eine konische Verjüngung (19) mit einem Innendurchmesser (21) von 6mm aufweist.

4. Soft-Laser nach einem der vorangehenden Ansprüche, ***dadurch gekennzeichnet,* dass** am vorderen Ende des flexiblen Schlauches (3) dieser über einen geriffelten Nippel (23) gesteckt ist, welcher Nippel (23) einstückig zu einem Einsatzstück (6) mit Fassung für den Endabschnitt der axial aus dem flexiblen Schlauch (3) herausragenden Fiberglasfaser (4) gehört, welches Einsatzstück (6) als Fassung eine innere axiale Bohrung von 2mm Durchmesser aufweist, zur Aufnahme der Fiberglasfaser (4) und an ihrem vorderen Ende in ihrem Inneren in eine sphärische Fläche (27) mit einem Radius von 2mm mündet, in welcher eine Licht-Streukugel (8) mit 4mm Durchmesser sitzt, die eine Sackbohrung (28) von 2mm Durchmesser zur Aufnahme der Fiberglasfaser (4) aufweist, und dass das Einsatzstück (6) ab dem Nippel (23) in einen verdickten Bereich (24) übergeht, welcher in axialer Richtung auf beiden Seiten je eine Schulter (25,26) bildet, und vor diesem verdickten Bereich (24) aussen nach vorne konisch in einem Winkel von 2° zuläuft und auf diesen Konus (22) ein zu ihm passendes Endstück (9) klemmend aufsteckbar ist.

5. Soft-Laser nach einem der vorangehenden Ansprüche, ***dadurch gekennzeichnet,* dass** am vorderen Ende des flexiblen Schlauches (3) dieser über einen geriffelten Nippel (23) gesteckt ist, welcher Nippel (23) einstückig zu einem Einsatzstück (6) mit Fassung für den Endabschnitt der axial aus dem flexiblen Schlauch (3) herausragenden Fiberglasfaser (4) gehört, welche Fassung eine innere axiale Bohrung von 2mm Durchmesser aufweist, zur Aufnahme der Fiberglasfaser (4), wobei die Fassung an ihrem vorderen Ende in eine sphärische Fläche (27) mit einem Radius von 2mm mündet, in welcher eine Licht-Streukugel (8) mit 4mm Durchmesser sitzt, die eine Sackbohrung (28) von 2mm Durchmesser zur Aufnahme der Fiberglasfaser (4) aufweist.
